# EUROPEAN PATENT APPLICATION

(11) **EP 1 112 735 A1**
(43) Date of publication of application: **04.07.2001**
(21) Application number: 00123522.5
(22) Date of filing: 27.10.2000
(51) Int. Cl.: A61K 7/032

(54) **Cosmetic composition applied to the eyelashes**

(30) Priority: 20.12.1999 US 467441
(71) Applicant: AVON PRODUCTS, INC., New York, NY 10020-1196 (US)
(72) Inventor: Mercado, Clara, Saddle River, NJ 07458 (US); Slobody, Brent, New City, NY 10956 (US)
(74) Representative: Dr. Weitzel & Partner

(57) **Abstract**

There is provided a cosmetic composition having an anti-static component that separates and defines the eyelashes, and optionally having a multipolymer system that curls the eyelashes and maintains the curl of the eyelashes.

## Description

### BACKGROUND OF THE INVENTION

### 1. Field of the Invention

The present invention relates to cosmetic compositions. More particularly, the present invention relates to cosmetic compositions for improving the separation, definition, and curling of eyelashes.

### 2. Background of the Invention

Mascara is a cosmetic composition used to enhance the beauty of a person's eyes by thickening, lengthening, coloring, curling, and defining the eyelashes.

Historically, mascara has been formulated as pigmented cakes having at least 50% soap. Cake mascara was applied to the lashes with a wet brush. The primary drawback of cake mascara is that the resulting film on the eyelashes is very water soluble and, thus, prone to smudging and running.

Presently, mascara is usually a cream emulsion-based product that uses a mixture of ingredients, such as waxes, polymers, pigments, and gums, among others. Cream emulsion-based mascara is popular since it is easy and more convenient to use. For example, cream emulsion-based mascara is typically contained in small tubes and applied with specially designed brushes. Cream emulsion-based mascara has surpassed cake mascara in popularity because of the above benefits, as well as the fact that cream emulsion-based mascara offers better aesthetics, and more water resistance.

However, cream emulsion-based mascara has limitations. First, such mascara that is viscous enough to uniformly coat the eyelashes tends to cause the eyelashes to stick together. Consumers find this effect very undesirable. Also, electrostatic attraction causes the eyelashes to stick together. The primary cause of electrostatic attraction between eyelashes is the charge of the iron or metal-oxide pigments in the mascara and the friction from repeatedly passing the mascara brush over the eyelashes.

There are prior art techniques for combating the tendency of mascara to stick eyelashes together. However, many prior art techniques focus on the applicator brush. In light of the foregoing, there remains a need for an improved cosmetic composition that separates and defines the eyelashes, as well as improves the curling and curl retention of the eyelashes.

### SUMMARY OF THE INVENTION

It is an object of the present invention to provide a cosmetic composition for application to the eyelashes.

It is another object of the present invention to provide such a cosmetic composition that optimizes, separates and curls the eyelashes.

It is still another object of the present invention to provide such a cosmetic composition that separates and defines the eyelashes while uniformly coating the eyelashes.

It is a further object of the present invention to provide such a cosmetic composition that provides curl and curl retention to the eyelashes.

### DETAILED DESCRIPTION OF THE INVENTION

The present invention is a cosmetic composition for application to the eyelashes, such as mascara. The mascara of the present invention has an anti-static component or ingredient that separates and defines the eyelashes. The mascara may also have a multipolymer system that curls the eyelashes and maintains the curl of the eyelashes.

The anti-static ingredient useful in the present composition includes a high molecular weight polysaccharide extracted from crustacean shells, quaternary ammonium compounds, amines, and betaines, and mixtures thereof. More preferably, the anti-static ingredient is chosen from one or more of the following: chitin, distearoylethyl hydroxyethylmonium methosulfate, acetamidopropyl trimonium chloride, behenyl betaine, dilaureth-4 dimonium chloride, dioctylamine, polyquaternium-10, PEG-2 soyamine, stearamidopropyl dimethylamine stearate, sodium lauriminodipropionate, PEG-5 stearyl ammonium chloride, and mixtures thereof.

The anti-static ingredient reduces electrostatic attraction between the individual eyelashes. Specifically, the residual positive charge of the anti-static ingredient effectively repels and separates the eyelashes from each other, while promoting an even, clump-free coating of the eyelashes. Additionally, the anti-static ingredient, with its high degree of deacetylation, proves an efficient film-former and substantive agent to the eyelashes. Furthermore, the anti-static ingredient may also have a residual negative charge, which provides conditioning benefits to the eyelashes.

A preferred anti-static ingredient is chitin. When used, the chitin is preferably a carboxy methyl chitin. It is more preferable to use a high molecular weight carboxy methyl chitin as the anti-static ingredient.

Carboxy methyl chitin has the following general structure:

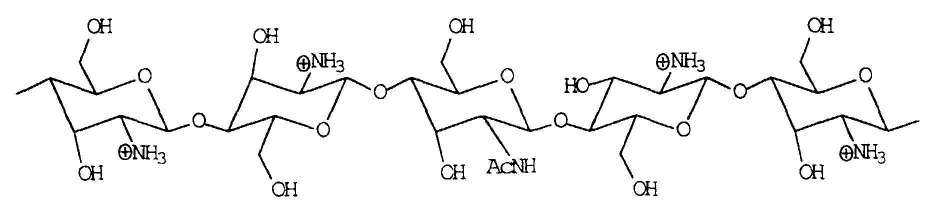

A preferred carboxy methyl chitin is supplied by Ichimara Pharcos Co. Ltd. of Japan.

Another preferred anti-static ingredient is a quaternary ammonium compound. Preferably, the quaternary ammonium compound may be a high molecular weight film former comprising an aqueous dispersion of non-phospholipid vesicles having a cationic surface charge and containing encapsulated jojoba oil, sweet almonic oil, wheat germ oil, apricot kernel oil, or mixtures thereof. This dispersion is preferably preserved with phenoxyethanol, methylparaben, benzoic acid, or mixtures thereof.

The anti-static ingredient is present in an amount about 0.1 percent by weight (wt%) to about 10 wt% of the total weight of the composition. Preferably, this ingredient is present in an amount about 0.5 wt% to about 5 wt%, and, more preferably, about 1 wt% to about 2.5 wt% of the total weight of the composition.

The multipolymer system for use in the present invention is a polymer complex, in particular a multipolymer resin, having a high polydispersity index (broad molecular weight and chain length distribution). Thus, the polymers of the multipolymer system may lay upon each other to bend the lash. This produces curling of the eyelash. It also maintains the curling of the eyelash.

The polymers useful in the multipolymer system include silicone-based, as well as vinyl-based, polymers and copolymers. More preferably, the polymers may be one or more of: dimethicone copolyol phosphate, polydimethylsiloxypropyl polyethoxy phosphate, polydimethylsiloxypropyl polyalkoxy phosphate, PVC/acrylates/lauryl methacrylate copolymer, PVM/MA copolymer, PVP/vinyl caprolactam/DMAPA acrylates copolymer, PVP/hexadecane copolymer, polyacrylamide, styrene-acrylate copolymer, acrylates copolymer, polyvinyl caprolactam, acrylates/succinates/hydroxyester polymer, decene butene copolymer, and polypropylene glycol 12 saturated methylene diphenyldiisocyanate copolymer. The more preferred polymers for use in the multipolymer system are the styrene-acrylate copolymers.

The multipolymer system is present in an amount about 0.5 wt% to about 30 wt% of the total weight of the composition. Preferably, the multipolymer system is present in an amount about 5 wt% to about 10 wt%, and more preferably about 5 wt% to about 8 wt% of the total weight of the composition.

In a preferred embodiment, the mascara is a cream emulsion-based composition that combines an anti-static component and a multipolymer system. In a more preferred embodiment, the cosmetic composition combines the anti-static component and the multipolymer system with one or more of the following: a chelating agent, an emulsifier, a film former (other than the antistatic ingredient or the multipolymer system), a gum, a moisturizer, water, and a wax or wax mixture. In a more preferred embodiment, the mascara is a cream emulsion-based composition that includes an emulsifier, a gum, water, and a wax mixture,

An emulsifier is defined as an ingredient that can suspend two more generally immiscible liquids in a stable mixture. In the present composition, any suitable emulsifier may be used. Such emulsifiers, include, but are not limited to, one or more soaps, phosphate esters, ethoxylated alcohols, ethoxylated fatty acids, ethoxylated fatty esters, polyol ether esters, glycerol esters, sucrose or sorbitan esters, glucose esters, potassium or DEA-cetyl phosphate, fatty esters, and mixtures thereof.

The amount of emulsifier in the present composition is preferably about 1 wt% to about 10 wt% based upon the total weight of the composition. The amount of emulsifier present in the composition is more preferably about 6 wt% to about 9 wt%, and most preferably about 5 wt% to about 8 wt% of the total weight of the composition.

A gum, as defined in this invention, is a complex and highly branched carbohydrate polymer, which is insoluble in alcohol and other organic solvents, but generally soluble or dispersible in water. A gum is a hydrophilic polysaccharide composed of monosaccharide units joined by glycosidic bonds. They occur as exudations from various trees and shrubs or as phytocolloids, and differ from natural resins in both chemical composition and solubility properties. A gum useful in the present composition can be arabic, acacia, carboxymethyl cellulose, hydroxyethyl cellulose, guar, tragacanth, or karaya, or mixtures thereof.

When one or more gums are used in the present composition, the total amount of gum present is preferably about 0.1 wt% to about 10 wt%. More preferably, the one or more gums are present in an amount about 0.5 wt% to about 8 wt%, and most preferably about 5 wt%.

A wax is a lower-melting point organic mixture, or a compound of high molecular weight, that is solid at room temperature. A wax is generally similar in composition to a fat and an oil, except that the carbon chain length of the wax is greater than 18.

The waxes that are useful in the present composition typically have a carbon chain length greater than about C₁₈ and a melting point about 50° to about 100°C. Such waxes may be a hydrocarbon, or an ester of fatty acid and alcohol. The one or more waxes or wax mixture that is useful in the present composition may be natural wax, which includes animal, vegetable, or petroleum, or may be a synthetic wax, which includes an ethylenic polymer, a polyol ether-ester, or a chlorinated naphthalene.

Preferred waxes useful in the present composition include beeswax, lanolin, shellac, carnauba, candelilla, bayberry, ozokerite, ceresin, paraffin, microcrystalline, polyethylene (ethylenic polymers), polyethylene, C₂₄₋₄₅ alkyl methicones, or mixtures thereof. More preferred waxes are beeswax, lanolin, carnauba, candelilla, ozokerite, ceresin, paraffin, microcrystalline, polyethylene, C₂₄₋₄₅ alkyl methicones, or mixtures thereof.

A wax is preferably present in an amount about 5 wt% to about 30 wt% of the total weight of the composition. More preferably, wax is present in an amount about 10 wt% to about 25 wt%, and most preferably about 13 wt% to about 25 wt% of the total weight of the composition.

A film former is a performance-enhancing ingredient that helps to ensure that the composition will not flake, transfer, run or smear. It also enhances the elongation and thickening of lashes, and coats and seals the powders and waxes in the composition.

The film former is preferably a polymer-based compound and/or a wax. Preferred film formers are polymer-based compounds having monomer units that include, but are not limited to, one or more olefin oxides, vinyl pyrrolidones, vinyl esters, vinyl alcohols, vinyl cyanides, oxazolines, carboxylic acids and esters, polysaccharides, urethanes, shellacs, or mixtures thereof. The amount of film former is preferably about 5 wt% to about 30 wt% of the total weight of the composition.

The present composition may also include a moisturizing agent. A moisturizing agent useful in the present composition includes one or more humectants. Examples of humectants suitable for use in the present composition include glycerin, sorbitol, propylene glycol, glycol dibehenate, glycol dioctanoate, glycol distearate, glycol hydroxystearate, glycol oleate, glycol ricinoleate, glycol salicyate, glycol stearate, glycol stearate DSE, sodium PCA, and mixtures thereof.

If used, a moisturizing agent is preferably present in an amount about 0.1 wt% to about 5 wt% of the total weight of the composition. Examples of moisturizing agents that may be used include hydrolyzed elastin, hydrolyzed keratin, hydrolyzed silk, hydrolyzed animal protein, hydrolyzed milk protein, hydrolyzed mucopolysaccharides, potassium coco-hydrolyzed animal protein, myristoyl hydrolyzed animal protein, and mixtures thereof.

The composition may also contain one or more chelating agents. The chelating agent is preferably present in an amount about 0.1 wt% to about 1.0 wt% of the total weight of the composition. The chelating agent is preferably disodium EDTA, trisodium EDTA, sodium hexametaphosphate or mixtures thereof.

There are many other ingredients that are useful in the present composition, which are traditionally incorporated into cream emulsion-based mascaras. Such ingredients include, but are not limited to, solids such as one or more pigments, fats, oils, gelatins, dyes, resins, fillers, thickeners, gelling agents, fragrances, odor adsorbers, preservatives, and mixtures thereof. Preferably, these other ingredients comprise about 20 wt% to about 30 wt%, and more preferably about 20 wt% to about 25 wt% of the total weight of the composition.

The pigments that may be used in the present composition can be inorganic, organic, or pearlescent. Such inorganic pigments include, but are not limited to, titanium dioxide, iron oxides, and mixtures thereof. Organic pigments and lakes useful in the present composition include, but are not limited to, FD&C Blue #1, FD&C Red No. 3, D&C Red No. 6, D&C Red No. 7, D&C Red No. 30, D&C Red No. 33, D&C Red No. 36, FD&C Yellow No. 5, FD&C Yellow No. 6, D&C Orange No. 4, D&C Orange No. 10, cochineal carmine, and mixtures thereof. Such pearlescent pigments include, but are not limited to, white pearlescent pigments such as, for example, mica coated with titanium oxide and bismuth oxychloride. In addition, colored pearlescent pigments may be used in the present composition such as, for example, chromium oxide, titanium mica with iron oxides, titanium mica with ferric blue, titanium mica with an organic pigment mentioned above as well as those based on bismuth oxychloride, and mixtures thereof. The pigments may optionally be surface-treated with, among other things, silicones, perfluorinated compounds, lecithin, and amino acids.

When employed, the pigments are present in proportions that depend on the intended color and intensity of the composition. The amount of pigment in a preferred composition, according to the present invention, is about 5 wt% to about 20 wt%, preferably about 6 wt% to about 15 wt%, and more preferably about 6 wt% to about 10 wt% of the total weight of the composition.

Fats useful in the present composition are generally defined as glyceryl esters of higher fatty acids such as, for example, stearic acid and palmitic acid. Such esters and their mixtures are solids at room temperature. Suitable fats that may be used in the present composition include fats derived from animals, vegetables, synthetically derived fats, or mixtures thereof. Preferred fats include glyceryl monostearate, glyceryl distearate, glyceryl tristearate, palmitate esters of glycerol, C₁₈₋₃₆ triglycerides, glyceryl tribehenate, or mixtures thereof.

Fillers useful in the present composition can be, for example, Teflon®, polymethacrylate, polyethylene, mica, talc, nylon, silica, kaolin, or mixtures thereof.

The cosmetic composition may also contain one or more preservatives, such as imidazolidinyl urea, diazolidinyl urea, methylparaben, ethylparaben, propylparaben, butylparaben, sodium dehydroacetate, sorbic acid, sodium benzoate, or mixtures thereof. The one or more preservatives are typically present in an amount about 1 wt% to about 3 wt%, and more preferably about 1 wt% to 2 wt% of the total weight of the composition.

The following is an example of a composition according to the present invention.

### EXAMPLE 1 - Mascara

| Wt% | Ingredient |
|---|---|
| 50 | Demineralized Water |
| 3 | Antistatic component including chitin and a cationic blend of jojoba, palm and vegetable oils |
| 6 | Multipolymer system including Black Iron Oxide/Styrene Acrylate Trtd. |
| 1 | Emulsifier including benzyl alcohol |
| 20 | Wax including beeswax, carnauba and paraffin |
| 4 | Gum |
| 8 | Structure agent such as fatty acid amines |
| 5 | Moisturizer, such as silicone |
| q.s. | Conventional chelating agent, preservative, vitamin, and conditioner |

The present invention having been described with particular reference to the preferred forms thereof, it will be obvious that various changes and modifications may be made herein without departing from the spirit and scope of the invention as defined in the appended claims. As used herein, singular can mean plural.

## Claims

1. A method of optimizing separation of eyelashes comprising the step of applying an anti-static ingredient to the eyelashes.

2. The method of claim 1, wherein said anti-static ingredient is selected from the group consisting of: high molecular weight polysaccharides extracted from crustacean shells, quaternary ammonium compounds, amines, betaines, and mixtures thereof.

3. The method of claim 2, wherein said anti-static ingredient is selected from the group consisting of: chitin, distearoylethyl hydroxyethylmonium methosulfate, acetamidopropyl trimonium chloride, behenyl betaine, dilaureth-4 dimonium chloride, dioctylamine, polyquaternium-10, PEG-2 soyamine, stearamidopropyl dimethylamine stearate, sodium lauriminodipropionate, PEG-5 stearyl ammonium chloride, and mixtures thereof.

4. The method of claim 3, wherein said anti-static ingredient is chitin.

5. The method of claim 4, wherein said anti-static ingredient is carboxymethyl chitin.

6. The method of claim 3, wherein said anti-static ingredient is stearamidopropyl dimethylamine stearate.

7. A method of optimizing curling of eyelashes comprising the step of:
applying a multipolymer system to the eyelashes, wherein said multipolymer system has a high polydispersity index.

8. The method of claim 7, wherein said multipolymer system comprises at least one polymer selected from the group consisting of silicone-based polymers, silicone-based copolymers, vinyl-based polymers, and vinyl-based copolymers.

9. The method of claim 8, wherein said multipolymer system comprises at least one polymer selected from the group consisting of: dimethicone copolyol phosphate, polydimethylsiloxypropyl polyethoxy phosphate, polydimethylsiloxypropyl polyalkoxy phosphate, PVC/acrylates/lauryl methacrylate copolymer, PVM/MA copolymer, PVP/vinyl caprolactam/DMAPA acrylates copolymer, PVP/hexadecane copolymer, polyacrylamide, styrene-acrylate copolymer, acrylates copolymer, polyvinyl caprolactam, acrylates/succinates/hydroxyester polymer, decene-butene copolymer, and polypropylene glycol 12 saturated methylene diphenyl diisocyanate copolymer.

10. The method of claim 9, wherein said multipolymer system comprises at least one polymer selected from the group consisting of: styrene-acrylate copolymer, decene butene copolymer, and acrylate/succinate/hydroxyester copolymer.

11. A method of optimizing separation and curling of eyelashes comprising the steps of:
applying an anti-static ingredient to the eyelashes; and
applying a multipolymer system to the eyelashes, wherein said multipolymer system has a high polydispersity index.

12. The method of claim 11, wherein said anti-static ingredient is selected from the group consisting of: high molecular weight polysaccharides extracted from crustacean shells, quaternary ammonium compounds, amines, betaines, and mixtures thereof.

13. The method of claim 12, wherein said anti-static ingredient is selected from the group consisting of: chitin, distearoylethyl hydroxyethylmonium methosulfate, acetamidopropyl trimonium chloride, behenyl betaine, dilaureth-4 dimonium chloride, dioctylamine, polyquaternium-10, PEG-2 soyamine, stearamidopropyl dimethylamine stearate, sodium lauriminodipropionate, PEG-5 stearyl ammonium chloride, and mixtures thereof.

14. The method of claim 13, wherein said anti-static ingredient is selected from the group consisting of stearamidopropyl dimethylamine stearate and chitin.

15. The method of claim 14, wherein said chitin is carboxymethyl chitin.

16. The method of claim 11, wherein said multipolymer system comprises at least one polymer selected from the group consisting of: silicone-based polymers, silicone-based copolymers, vinyl-based polymers, and vinyl-based copolymers.

17. The method of claim 16, wherein said multipolymer system comprises at least one polymer selected from the group consisting of: dimethicone copolyol phosphate, polydimethylsiloxypropyl polyethoxy phosphate, polydimethylsiloxypropyl polyalkoxy phosphate, PVC/acrylates/lauryl methacrylate copolymer, PVM/MA copolymer, PVP/vinyl caprolactam/DMAPA acrylates copolymer, PVP/hexadecane copolymer, polyacrylamide, styrene-acrylate copolymer, acrylates copolymer, polyvinyl caprolactam, acrylate/succinate/hydroxyester polymer, decene-butene copolymer, and polypropylene glycol 12 saturated methylene diphenyl diisocyanate copolymer.

18. The method of claim 17, wherein said multipolymer system comprises at least one polymer selected from the group consisting of: styrene-acrylate copolymer, decene-butene copolymer, and acrylate/succinate/hydroxyester polymer.

19. The method of claim 11, wherein said anti-static ingredient comprises chitin and said multipolymer system comprises styrene-acrylate copolymer.

20. A cosmetic composition for application to eyelashes comprising:
an anti-static component, wherein said anti-static component is selected from the group consisting of: high molecular weight polysaccharides extracted from crustacean shells, quaternary ammonium compounds, amines, betaine, and mixtures thereof; and
a multipolymer system having a high polydispersity index, wherein said multipolymer system comprises at least one polymer selected from the group consisting of: silicone-
based polymers and copolymers, and vinyl-based polymers and copolymers,
wherein said anti-static ingredient separates and defines the eyelashes, and wherein said multipolymer system curls the eyelashes.

21. The composition of claim 20, wherein said anti-static compound is about 0.1 wt% to about 10 wt% of the total weight of the composition, and wherein said multipolymer system is about 0.5 wt% to about 30 wt% of the total weight of the composition.

22. The method of claim 20, wherein said anti-static ingredient is selected from the group consisting of: chitin, distearoylethyl hydroxyethylmonium methosulfate, acetamidopropyl trimonium chloride, behenyl betaine, dilaureth-4 dimonium chloride, dioctylamine, polyquaternium-10, PEG-2 soyamine, stearamidopropyl dimethylamine stearate, sodium lauriminodipropionate, PEG-5 stearyl ammonium chloride, and mixtures thereof.

23. The method of claim 22, wherein said anti-static ingredient is selected from the group consisting of stearamidopropyl dimethylamine stearate and chitin.

24. The method of claim 20, wherein said multipolymer system comprises at least one polymer selected from the group consisting of: dimethicone copolyol phosphate, polydimethylsiloxypropyl polyethoxy phosphate, polydimethylsiloxypropyl polyalkoxy phosphate, PVC/acrylates/lauryl methacrylate copolymer, PVM/MA copolymer, PVP/vinyl caprolactam/DMAPA acrylates copolymer, PVP/hexadecane copolymer, polyacrylamide, styrene-acrylate copolymer, acrylates copolymer, polyvinyl caprolactam, acrylate/succinate/hydroxyester polymer, decene-butene copolymer, and polypropylene glycol 12 saturated methylene diphenyl diisocyanate copolymer.

25. The method of claim 24, wherein said multipolymer system comprises at least one polymer selected from the group consisting of: styrene-acrylate copolymer, decene-butene copolymer, and acrylate/succinate/hydroxyester polymer.

26. The composition of claim 26, further comprising a wax, an emulsifier, a film former, a chelating agent, and a pigment.
